(19) Europäisches Patentamt
European Patent Office
Office européen des brevets

(11) Publication number: **0 423 333 A1**

(12) # EUROPEAN PATENT APPLICATION published in accordance with Art. 158(3) EPC

(21) Application number: 89905185.8

(22) Date of filing: 01.05.89

(86) International application number: PCT/JP89/00455

(87) International publication number: WO 89/11099 (16.11.89 89/27)

(51) Int. Cl.[5]: G01N 33/53, G01N 33/577

(30) Priority: 07.05.88 JP 110938/88
28.05.88 JP 131086/88

(43) Date of publication of application: 24.04.91 Bulletin 91/17

(84) Designated Contracting States: AT BE CH DE FR GB IT LI LU NL SE

(71) Applicant: Yamasa Shoyu Kabushiki Kaisha
10-1, Araoi-Cho 2-Chome
Choshi-Shi Chiba-Ken 288(JP)

(72) Inventor: KIMURA, Sadao
1867-207-105, Namiki 2-chome
Tsukuba-shi, Ibaraki-ken 305(JP)
Inventor: YANAGISAWA, Masashi
8-14-907-303, Takezono 1-chome
Tsukuba-shi, Ibaraki-ken 305(JP)
Inventor: YAZAKI, Yoshio
12-4, Kobinata 3-chome
Bunkyo-ku Tokyo-to 112(JP)
Inventor: KURIHARA, Hiroki,Room 904, Kameidotenjinmae-Sky Mansion, 3-6, Kameido 3-chome, Koto-ku
Tokyo-to 136(JP)
Inventor: HAMAOKI, Masaru
2-2, Sakae-cho 2-chome
Choshi-shi Chiba-ken 288(JP)
Inventor: KATO, Hirohisa
2-2, Sakae-cho 2-chome
Choshi-shi Chiba-ken 288(JP)

(74) Representative: Dr. Elisabeth Jung Dr. Jürgen Schirdewahn Dipl.-Ing. Claus Gernhardt
P.O. Box 40 14 68 Clemensstrasse 30
W-8000 München 40(DE)

(54) ## ANTI-ENDOSERINE ANTIBODY.

(57) An antibody specifically reacting with endoserine, a process for its preparation, an immunological assay of endoserine using said antibody as an antibody reagent, and a kit for the assay are disclosed.

EP 0 423 333 A1

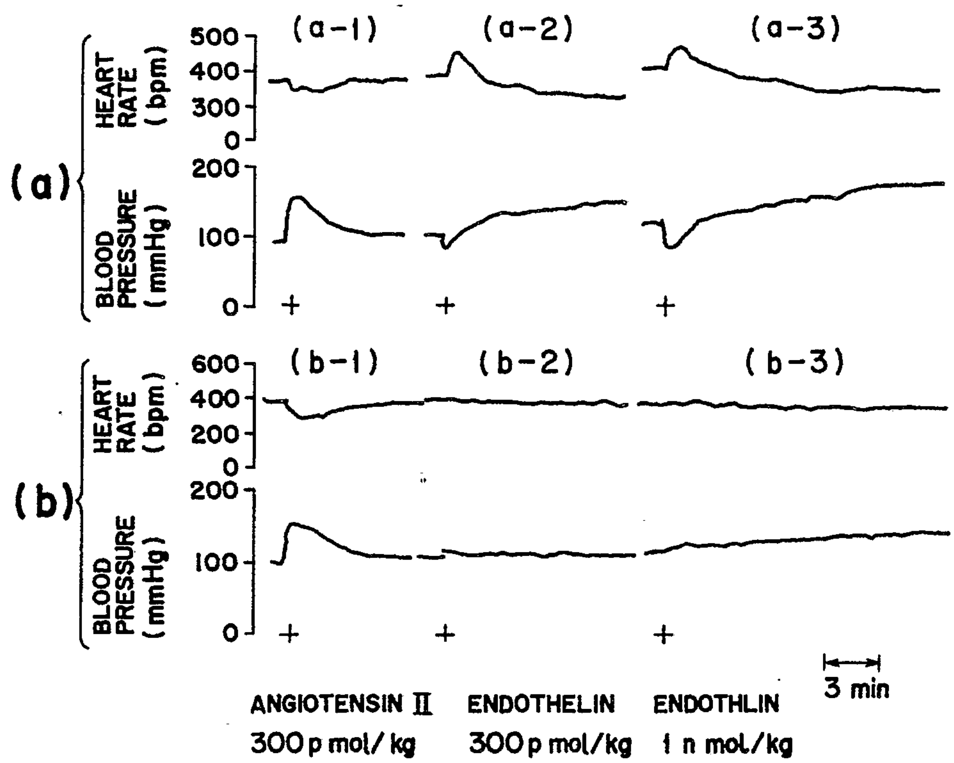
(a-1)
(a-2)
(a-3)
(b-1)
(b-2)
(b-3)
(a)
(b)
HEART RATE (bpm)
BLOOD PRESSURE (mmHg)
500
400
300
0
200
100
0
600
400
200
0
200
100
0
3 min
ANGIOTENSIN II 300 p mol/kg
ENDOTHELIN 300 p mol/kg
ENDOTHLIN 1 n mol/kg

FIG. 4

# ANTI-ENDOTHELIN ANTIBODY

## TECHNICAL FIELD

This invention relates to an antibody having specificity for endothelin, a method for preparing said antibody, and a method for immunological assay of endothelin and a kit for immunological assay of endothelin by the use of said antibody.

## BACKGROUND ART

Endothelin is a novel physiologically active peptide having vasoconstrictive activity and is secreted from vascular endothelial cells (see Nature, Vol. 332, p. 441-415 (1988)). It has been considered that assaying the level in blood of this physiologically active peptide, its fragments obtained by hydrolysis, or its precursor is significant for clarification of the mechanism of essential hypertension.

In the prior art, as a means for assaying the concentrations of physiologically active peptides such as hormones, various immunological assay methods have been developed [see Naibunpi Jikken Koza (Experiment Lectures For Internal Secretions), Vol. 5, "Hormone Sokuteiho (Jo) (Hormone Assay - Part I)", ibid. Vol. 6 "Hormone Sokuteiho (Ge) (Hormone Assay - Part II)" (all published by Kodansha K.K., Tokyo, on May 20, 1982)]. However, in the case of endothelin, no antibody having specificity for endothelin has been so far prepared, and for this reason, it has been impossible to assay endothelin according to the immunological assay method.

Accordingly, a first object of the present invention is to obtain an antibody having specificity for endothelin, and next a second object of the present invention is to establish a method for assaying immunologically endothelin by the use of the antibody obtained.

## DISCLOSURE OF THE INVENTION

The present inventors have made extensive studies in order to obtain an antibody having specificity for endothelin, and consequently obtained successfully said antibody and also found that endothelin can be assayed by the use of the antibody obtained, whereby the present invention has been accomplished.

Thus, the present invention provides an antibody having specificity for endothelin (hereinafter sometimes referred to as the antibody of the present invention).

Also, the present invention provides a method for preparing an antibody having specificity for endothelin.

Also, the present invention provides a method for immunological assay of endothelin by the use of an antibody having specificity for endothelin.

Further, the present invention provides a kit for immunologically assaying endothelin.

## BRIEF DESCRIPTION OF THE DRAWINGS

Figs. 1, 2, and 3 are graphs each showing the relationship between dilution ratio of an antiserum and its absorbance. Fig. 1 has been prepared by the use of an antiserum obtained by immunizing mouse with the antigen comprising a conjugate of human endothelin and bovine serum albumin. Fig. 2 has been prepared by the use of an antiserum obtained by immunizing rabbit with the antigen comprising a conjugate of endothelin derived from a human and egg albumin. Fig. 3 has been prepared by the use of an antiserum obtained by immunizing mouse with the antigen comprising a conjugate of a synthetic peptide as described later and bovine serum albumin.

Fig. 4 is a graph showing the neutralization action on endothelin of the anti-endothelin monoclonal antibody of the present invention. Fig. 4(a) shows the results when no monoclonal antibody of the present invention is administered, and Fig. 4(b), the results when the monoclonal antibody of the present invention is administered.

Figs. 5, 6, and 7 are calibration curves prepared by the use of the anti-endothelin monoclonal antibody of the present invention. In the Figures, filled circles (●) indicate values obtained when a solution dissolving human endothelin is used as an antigen solution, and open circles (O), values obtained when a solution

dissolving a synthetic peptide as described later is used as an antigen solution, respectively. The lines above and below the filled and open circles represent the standard deviation when the same measurement is conducted 5 times. Fig. 5 has been prepared by the use of a monoclonal antibody having specificity for a site other than the C-terminus of the endothelin produced by the 17th hybridoma. Fig. 6 and Fig. 7 have been prepared by the use of monoclonal antibodies having specificity for the C-termini of endothelins produced by the 16th hybridoma and the 20th hybridoma, respectively.

Fig. 8 is a calibration curve prepared according to the sandwich method using the two kinds of monoclonal antibodies produced by the first hybridoma and the 5th hybridoma.

## BEST MODE FOR PRACTICING THE INVENTION

The present invention is described in detail below.

### I. Preparation of Antigen

Examples of the antigen to be used for preparation of the antibody of the present invention are endothelins prepared from mammals such as a human, monkey, pig, bovine, goat, rabbit, guinea pig, rat, and mouse, fragments obtained by hydrolysis of these endothelins, or synthetic peptides having one kind or more kinds of the same antigenic determinants as these endothelins.

As a specific example of the endothelin prepared from a mammal, an endothelin derived from a human or pig having the amino acid sequence of the following formula [I] can be exemplified, the endothelin derived from a human and that derived from pig having the same structure:

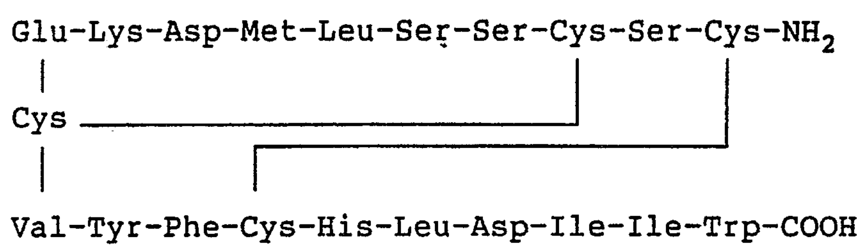

[I]

[wherein Cys represents L-cysteine; Ser, L-serine; Leu, L-leucine; Met, L-methionine; Asp, L-aspartic acid; Lys, L-lysine; Glu, L-glutamic acid; Val, L-valine; Tyr, L-tyrosine; Phe, L-phenylalanine; His, L-histidine; Ile, L-isoleucine; Trp, L-tryptophan].

As a fragment obtained by hydrolysis of endothelin, examples are fragments obtained by hydrolysis with various enzymes, such as fragments obtained by successively cleaving an endothelin derived from a human or pig as mentioned above from the N-terminus side and/or the C-terminus side by the use of exopeptidases such as aminopeptidase and carboxypeptidase, and fragments obtained by cleavage with endopeptidases such as serine protease, thiol protease, carboxyl protease and metal protease.

As a synthetic peptide, for example, synthetic peptides containing one kind or more kinds of the same amino acid sequence as that at any site comprising 3 or more, preferably 6 or more amino acids in the amino acid sequence of the above-mentioned human or porcine endothelin can be exemplified.

Such endothelins, their fragments obtained by hydrolysis, and synthetic peptides having one kind or more kinds of the same antigenic determinants as those existing in endothelin (hereinafter these being referred to merely as "endothelin antigen") may be prepared by adequately choosing the best preparation method corresponding to the kind of the objective endothelin antigen from conventional methods such as the method of isolating it from the conditioned medium of vascular endothelial cells and the chemical synthetic method.

As the method for isolating the endothelin antigen from the conditioned midium of vascular endothelial cells, for example, the culture supernatant of the endothelial cells obtained by the scraping method from the aorta of mammals such as pig, can be treated by a suitable combination of conventional isolation and purification means of peptides such as ion exchange chromatography and reverse phase chromatography, whereby the endothelin antigen can be isolated and purified (for details, see Nature, Vol. 332, 411-415 (31, March, 1988); Jikken Igaku (Experimental Medicine), Vol. 6 (No. 4), p. 11-16 (1988)).

The chemical synthetic method of the endothelin antigen may be practiced by suitably choosing a

method from among those conventionally used, such as the azide method, the acid chloride method, the asymmetric acid anhydride method (the mixed acid anhydride method, the phosphoric acid or arsenic acid anhydride method), the symmetric acid anhydride method (N,N'-dicyclohexylcarbodiimide (DCC) method), the active ester method (the p-nitrophenyl ester method, the N-hydroxysuccinimide ester method, and the cyanomethyl ester method), the method using Woodward reagent K, the N, N'-carbonyldiimidazole method, the redox method, and the DCC/additive (e.g. HONB, HOBT, HOSu) method. These chemical synthetic methods may be either the liquid phase method or the solid phase method, and, as the synthesis procedure, either the stepwise elongation method in which amino acids are condensed one by one, or the method of the fragment condensation method in which fragments comprising several amino acids are condensed. As to specific procedures and techniques of these synthetic methods, reference can be made to textbooks ["The Peptides" Vol. 1, Academic Press, New York, USA (1966); "Peptide Synthesis" (edited by Maruzen Kabushiki Kaisha, Japan, 1975)].

The endothelin antigen thus obtained has a low molecular weight and a low antigenicity, and therefore a conjugate having it bound to or adsorbed on an appropriate high molecular weight carrier is used as an immunogen.

As the high molecular weight carrier for binding or adsorbing the endothelin antigen, natural or synthetic carriers conventionally used in preparation of antibodies against hapten antigens can be used. Examples of natural high molecular weight carriers include animal serum albumins such as bovine serum albumin, rabbit serum albumin and human serum albumin; animal serum globulins such as bovine serum globulin, rabbit serum globulin, human serum globulin and goat serum globulin; animal thyroglobulins such as bovine thyroglobulin and rabbit thyroglobulin; animal hemoglobins such as bovine hemoglobin, sheep hemoglobin and human hemoglobin; and hemocyanins such as keyhole limpet hemocyanin. Examples of synthetic high molecular weight carriers are various latices of polymers or copolymers of amino acids such as polylysine, polyglutamic acid and lysine-glutamic acid copolymer; and of polymers or copolymers prepared from aromatic vinyl compounds, $\alpha,\beta$-unsaturated carboxylic acids or esters thereof, $\alpha,\beta$-unsaturated nitrile compounds, vinyl halide compounds and conjugated diene compounds, such as styrene, chlorostyrene, $\alpha$-methylstyrene, divinylbenzene, sodium styrenesulfonate, (meth)acrylic acid, ethyl(meth)acrylate, (meth)-acrylonitrile, (meth)acrolein, (meth)acrylamide, butadiene, isoprene, vinyl acetate, vinylpyridine, N-vinyl-2-pyrrolidone, vinyl chloride and vinylidene chloride.

For binding of such a carrier with the endothelin antigen, any binding method of the physical adsorption method, the covalent bonding method, the ionic bonding method, etc. may be employed.

The physical adsorption method can be practiced, for example, by stirring polyvinyl pyrrolidone or latex particles with the endothelin antigen slowly at room temperature for 1 to 3 hours (for details, see Endocrinology, 93 , 1092 (1973); Japanese Laid-Open Patent Publication No. 43124/1986, etc.). The covalent bonding method can be practiced by using 1 to 50-fold moles of the endothelin antigen per mole of a high molecular weight carrier and an excessive amount of a binding reagent in various buffers such as borate buffer and phosphate buffer, in the presence of a binding agent which can bind the endothelin antigen with the carrier by activating various functional groups existing in the endothelin antigen or the carrier such as amino group, carboxyl group and sulfhydryl group, and carrying out the reaction at a reaction temperature of 0 to 50 °C for 30 minutes to 30 hours.

As the binding agent to be used for binding the endothelin antigen with the carrier, those conventionally used in binding between a hapten or peptide and a carrier or enzyme may be employed. For example, bisdiazonium compounds such as bisdiazotized benzidine and bisdiazotized 3,3'-dianisidine, which cause crosslinking the heterocyclic rings in tyrosine, histidine and tryptophan; dialdehyde compounds such as glyoxal, malondialdehyde, glutaraldehyde, succinaldehyde and adipaldehyde, diisocyanate compounds such as toluene-2,4-diisocyanate and xylenediisocyanate, halonitrobenzene compounds such as 2,4-dinitro-1,5-difluorobenzene and p,p'-difluoro-m,m'-dinitrophenylsulfone, and imide ester compounds such as diethylmalonimidate, all of which cause crosslinking among amino groups; dimaleimide compounds such as N,N'-o-phenylene dimaleimide and N,N'-m-phenylene dimaleimide, which cause crosslinking among thiol groups; maleimide succinimide compounds such as N-(m-maleimidobenzoyloxy)succinimide, 4-(maleimidomethyl) benzoic acid-N-hydroxysuccinimide ester, m-maleimidobenzoyl-N-hydroxysuccinimide ester and 4-(maleimidomethyl)-cyclohexane-1-carboxyl-N'-hydroxysuccinimideester, which cause crosslinking between amino group and thiol group; carbodiimide compounds such as N,N'-dicyclohexylcarbodiimide, N-ethyl-N'-dimethylaminocarbodiimide, 1-ethyl-3-diisopropylaminocarbodiimide and 1-cyclohexyl-3-(2-morpholinyl-4-ethyl)carbodiimidomethyl-p-toluene sulfonate, isoxazolium salt compounds such as N-ethyl-5-phenylisoxazolium-3'-sulfonate, and alkylchloroformate compounds such as ethyl chloroformate and isobutyl chloroformate, all of which cause crosslinking between amino group and carboxyl group can be used.

These binding agents are employed by choosing them depending on the kinds of the functional group

in the endothelin antigen and the functional group in the carrier. As the functional group in the endothelin antigen, for example, in the case of the endothelin represented by the above formula [I] derived from a human or pig, heterocyclic rings of tyrosine, tryptophan and hystidine, carboxyl groups of aspartic acid, glutamic acid and the C-terminus, amino group of the N-terminus or lysine, thiol group of cysteine, etc. are examples.

The immunogen thus obtained (conjugate of endothelin antigen and carrier) can be isolated and purified according to any of the conventional methods such as the dialysis method and the gel filtration method.

Among the immunogens prepared as described above, those having 2 to 20-fold moles of the endothelin antigen bound per mole of the carrier are particularly useful for preparation of the antibody having specificity for endothelin.

II. Preparation of Antibody

When the antibody is to be prepared by the use of the immunogen as described above, it can be prepared according to the conventional method in which the immunogen is administered to an animal to produce the antibody having specificity for endothelin in a living body and the antibody is collected.

More specifically, examples of the animal to which the immunogen is administered are mammals such as bovine, horse, sheep, rat, mouse, guinea pig, dog, pig, rabbit and monkey, and birds such as pigeon and chicken. Particularly convenient are mouse, rat, guinea pig and rabbit.

The immunogen is administered to such animals in a conventional manner. For example, a suspension of an adjuvant such as complete Freund's adjuvant, incomplete Freund's adjuvant, alum adjuvant, aluminum hydroxide adjuvant or pertussis adjuvant with the above-described immunogen is prepared and injected intravenously, intraperitoneally, subcutaneously or intradermally to the animals mentioned above.

The dosage is of the order of 0.01 to 10 mg/head as the amount of antigen when using rabbit or guinea pig as the animal, or of the order of 0.001 to 1 mg/head when using mouse or rat.

After the first administration, a similar booster as described above can be administered about cnce to 5 times every 1 to 4 weeks to obtain an antibody having specificity for endothelin.

The antibody thus prepared can be obtained as antiserum containing the antibody having specificity for endothelin as the main ingredient by collecting the blood 1 to 2 weeks after the final booster and subjecting the blood to centrifugation. When purification of the antibody is required, after fractionating only the antibody having specificity only for endothelin by utilizing an immobilized antigen obtained by immobilization of the antigen mentioned above, the antibodies existing in the antiserum may be also purified by fractionation for the respective classes of the antibodies according to a suitable combination of conventional methods such as the selective fractionation utilizing solubility difference (e.g., salting out and alcohol precipitation), the fractionation utilizing charge difference (e.g., ion exchange chromatography and electrophoresis) and the fractionation utilizing molecular weight difference (e.g., ultracentrifugation method and gel filtration method).

Next, as to the preparation of the monoclonal antibody having specificity for endothelin, this monoclonal antibody can be prepared by applying suitably any of the cell fusion methods, the transformation methods with EB virus, etc., known in the art.

Referring to the cell fusion method suitable for bulk production of monoclonal antibodies as an example, the monoclonal antibody having specificity for endothelin can be obtained according to the procedure as described below.

a) Preparation of antibody-producing cells:

The above mentioned immunogen is administered to the same animal as described above under the same conditions, and the antibody-producing cells (spleen cells, lymph node cells or peripheral blood cells) are obtained in a conventional manner from the animal which has acquired immunity.

b) Preparation of myeloma cells:

As myeloma cells, cell lines derived from various animals such as mouse, rat, rabbit and human and generally available to those skilled in the art are employed. Preferably, the cell line to be used should have drug resistance and have the properties of being not viable in a selective medium under unfused state, but viable only under the state fused with the antibody-producing cells. Generally, 8-azaguanine resistant cell line is used, and this cell line is defective in hypoxanthine guanine phosphoribosyltransferase and cannot grow in hypoxanthine-aminopterin-thymidine (HAT) medium. Also, as a property of the cell, it is preferable that the cell line be of the so-called non-secretor type which secrets no immunoglobulin.

Specific examples of myeloma cell lines are mouse myeloma cell lines such as P3X63Ag8 (ATCC

TIB-9) (Nature, 256 , 495-497 (1975)), P3X63Ag8 U.I ($P_3U_1$) (ATCC CRL-1597) (Current Topics in Microbiology and Immunology, 81 , 1-7 (1978)), P3X63Ag8. 653 (ATCC CRL-1580) (J. Immunology, 123 , 1548-1550 (1979), P3/NSI/l-Ag4-l (ATCC TIB-18) (European J. Immunology, 6 , 511-519 (1976)), and Sp2/O-Ag14 (ATCC CRL-1581) (Nature, 276 , 269-270 (1978)); rat myeloma cell lines such as 210. RCY. Ag 1.2.3 (Y3-Ag1.2.3) (ATCC CRL-1631) (Nature, 277 , 131-133 (1979)); human myeloma cell lines such as U-266-$AR_1$ (Proc. Natl. Acad. Sci. U.S.A., 77 , 5429 (1980)), GMI500 (Nature, 288 , 488 (1980)) and KR-4 (Proc. Natl. Acad. Sci. U.S.A., 79 , 6651 (1982)).

c) Cell fusion:

For cell fusion, myeloma cells compatible with the antibody-producing cells are selected. Cell fusion can be practiced by mixing $10^7$ to $10^8$ myeloma cells with antibody-producing cells at a mixing ratio of 1:4 to 10 in a medium for culturing animal cells such as Eagle's minimum essential medium (MEM), Dulbecco's modified Eagle's medium (DMEM) and RPMI 1640 medium. For promoting cell fusion, a fusogen such as a polyethylene glycol (PEG) having an average molecular weight of 1,000 to 6,000, a polyvinyl alcohol or Sendai virus can be used.

Also, by means of a commercially available cell fusion device utilizing electric pulses, the antibody-producing cells and the myeloma cells can be fused together.

d) Selection of hybridoma in selective medium:

As the method for selecting the desired hybridoma from the cells after cell fusion treatment, the method of utilizing selective proliferation of cells in a selective medium can be employed. For example, after diluting appropriately the cell liquid with RPMI 1640 medium containing 15% fetal calf serum (FCS), etc., the diluted suspension is seeded at about $10^5$ to $10^6$ cells/well on a microplate, and a selective medium (e.g., HAT medium) is added into each well, which step is followed by culturing with appropriate exchange of the selective medium. When 8-azaguanine resistant cell line is used as the myeloma cell and a HAT medium as the selective medium, unfused myeloma cells will die by about 10 days after cultivation. Also antibody-producing cells which are normal cells cannot grow in vitro for a long time, and therefore the cells grown on the 10th to 14th day after cultivation can be obtained as hybridomas.

e) Screening of hybridomas producing monoclonal antibody having specificity for endothelin:

Screening of the hybridomas producing the monoclonal antibody having specificity for endothelin can be practiced according to, for example, the enzyme-linked immunosorbent assay (ELISA), the radioimmunoassay (RIA). For example, into a 96-well microplate for ELISA having the endothelin antigen adsorbed thereon is added a culture supernatant containing the monoclonal antibody to allow the specific antibody to react with the endothelin antigen, and then the bound specific antibody is allowed to react with an enzyme-labeled antiimmunoglobulin antibody, or with a biotin-labeled antiimmunoglobulin antibody and thereafter avidin D-enzyme-labeled material, which step is followed by addition of an enzyme substrate into each well to cause color formation. According to the presence or absence of color formation, screening of the hybridoma producing an antibody having binding activity to endothelin can be carried out.

f) Cloning:

Cloning of the hybridomas can be practiced according to, for example, the limiting dilution method, the soft agar method, the fibrin gel method and the fluorescence-activated cell sorter method.

g) Production of monoclonal antibody:

As the method for producing the anti-endothelin monoclonal antibody from the hybridoma thus obtained, a conventional cell cultivation method or ascites formation method can be employed.

In the cell cultivation method, the hybridoma is cultured in a medium for culturing animal cells such as RPMI 1640 medium containing 10 to 15% FCS or serum-free medium, and the antibody can be obtained from the culture supernatant.

In the method of recovering the antibody from ascites, after a mineral oil such as pristane (2,6,10,14-tetramethylpentadecane) has been administered intraperitoneally into an animal the major histocompatibility of which coincides with the hybridoma, the hybridoma is intraperitoneally administered in an amount of about $10^7$ cells. Hybridomas will form ascitic tumors within about 10 to 18 days to produce antibodies at a high concentration in serum and ascites.

When purification of the antibody is required, it can be carried out by selecting and combining suitably known methods such as the ammonium sulfate salting-out method and various chromatographies such as ion exchange chromatography utilizing anion exchanger such as DEAE cellulose, affinity chromatography using Protein A-Sepharose and molecular sieve chromatography.

## III. Immunological Assay for Endothelin in Test Sample

The specific feature of the assay of the present invention resides in the use of the antibody of the present invention or active fragments thereof [e.g., $F(ab')_2$, Fab' and Fab] as the antibody reagent. It is not limited by the system itself of the immunological assay employed. More specifically, any assay can be employed as the assay of the present invention provided that it is a method in which the antigen-antibody reaction is carried out by utilizing the binding ability of the antigen to be assayed with the antibody which reacts specifically therewith, the amount of the antibody, the antigen, or the antibody-antigen conjugate corresponding to the amount of antigen existing in the test sample after the reaction being assayed by chemical or physical means, and the amounts of antigen corresponding to the measured values being calculated from a standard curve prepared by the use of the standard solutions containing given amounts of the antigen.

Examples of such an assay are nephelometry (e.g., the immunonephelometric method utilizing agglutination inhibition reaction, the immunoturbidimetric assay), the competitive reaction method (e.g., the liquid phase method and the solid phase method), the immunometric assay and the sandwich assay. As the labeled substance to be used in the assay system, radioisotopes (e.g., $^{125}I$, $^{131}I$, $^{3}H$ and $^{14}C$), enzymes (e.g., $\beta$-galactosidase, peroxidase, alkaline phosphatase and glucose-6-phosphate dehydrogenase), coenzyme/prosthetic groups (e.g., FAD, FMN, ATP, biotin and heme), fluorescent dyes [fluorescein derivatives (e.g., fluorescein isothiocyanate and fluorescein thioflubamyl), rhodamine derivatives (e.g., tetramethyl-rhodamine B isothiocyanate), umbelliferone and 1-anilino-8-naphthalenesulfonic acid], luminol derivatives (e.g., luminol, isoluminol and N-(6-aminohexyl)-N-ethylisoluminol), nitro oxide radical (e.g., 3-substituted-2,2,5,5-tetramethylpyrrolidine-1-oxyl), and metal complexes are examples.

When these individual immunological assays are applied to the method of the present invention, special conditions, operations, etc. need not be set. The assaying system may be constituted so that the endothelin to be assayed can be assayed with good sensitivity by the use of the antibody of the present invention or active fragments thereof as the antibody reagent in the method employed with conventional technical considerations of those skilled in the art in addition to conventional conditions and means in the respective methods. As to details relating to the general techniques of these methods, reference can be made to general reviews, textbooks, etc. [for example, "Radioimmunoassay" edited by Minoru Irie (Kodansha K.K., published on April 10, 1974); Radioimmunoassay, second series" edited by Minoru Irie (Kodansha K.K., published on May 1, 1979); "Enzyme Immunoassay" edited by Eiji Ishikawa et al. (Igaku Shoin K.K., published on December 15, 1978); "Enzyme Immunoassay" (2nd ed.) edited by Eiji Ishikawa et al. (Igaku Shoin K.K., December 15, 1982); Rinshobyori (Clinical Pathology), Supplemental Vol. 53, "Immunoassay for clinical tests - Techniques and Applications" (Rinshobyori Kankokai, published in 1983); "New Applied Cases of Immunoassay and Application to Developments of Diagnostic Reagents and Therapy - Collection of Overall Technical Information" (Keiei Kyoiku Shuppan, published on June 20, 1985); Yuki Gosei Kagaku (Organic Synthetic Chemistry), Vol. 38, No. 2 (1980), p. 151-163; "Methods in Enzymology", Vol. 70 (Immunochemical Techniques (Part A)); the same book Vol. 73 (Immunochemical Techniques (Part B)); the same book Vol. 74 (Immunochemical Techniques (Part C)); the same book Vol. 84 (Immunochemical Techniques (Part D: Selected Immunoassay)); and the same book Vol. 92 (Immunochemical Techniques (Part E: Monoclonal Antibodies and General Immunoassay Methods)) (all published by Academic Press Co.)].

Of these immunoassay methods, representative assay systems are as described below.

(1) Competitive reaction method:

The antigen in a test sample and a predetermined amount of a labeled antigen are subjected to a competitive reaction with an antibody; the unreacted antigen (F) and the antigen (B) bound to the antibody are separated from each other (B/F separation); and the label amount of either one of B and F is measured to determine quantitatively the antigen amount in the test sample.

For the above reaction method, there are the liquid phase method, in which a soluble antibody is used as an antibody and polyethylene glycol and a second antibody specific to the above antibody are used for B/F separation, and the solid phase method, in which solid phase antibody is used as an antibody, or a soluble antibody is used as a first antibody and a solid phase antibody is used as a second antibody.

(2) Immunometric assay:

After a competitive reaction of the antigen in a test sample and a solid phase antigen against a predetermined amount of a labeled antibody, the solid phase and the liquid phase are separated from each other; or, after the reaction of the antigen in a test sample and an excessive amount of a labeled antibody, a solid phase antigen is added to cause to react the unreacted labeled antibody therewith, which step is followed by separation of the solid phase and the liquid phase; and the label amount in either one of the phases is measured to quantitate the antigen in the test sample.

(3) Sandwich assay:

The antigen in a sample is allowed to react with a first antibody formed into solid phase and further allowed to react with a labeled second antibody which recognizes a determinant different from that for the first antibody, which step is followed by separation of the liquid phase and the solid phase; and the label amount in the liquid phase or the solid phase is measured to quantitate the antigen amount in the sample. In the above assay, both a polyclonal antibody and a monoclonal antibody can be used as the first antibody or the second antibody, and a combination of a polyclonal antibody as the first antibody and a monoclonal antibody as the second antibody, or a reversed combination thereof may also be used.

As a modified method, there is also an indirect labeling method in which, without labeling directly the second antibody, an antigen different from the antigen to be assayed or a binding member (a compound such as biotin) bound to the second antibody is used; after completion of the reaction between the second antibody and the antigen to be measured, a labeled material of a third antibody specific to the antigen bound to the second antibody or of a bindable substance specific to the binding member (avidin, etc.) is allowed to react with the reaction mixture, which step is followed by separation of the liquid phase and the solid phase for quantitation of the label amount in either one of the phases. As a combination of the binding member and the bindable substance, a biotin-avidin system has been widely employed.

IV. Endothelin Assay Reagents Kit

The kit of reagents for assay of the present invention is characterized by the use of the antibody of the present invention or active fragments thereof as an antibody reagent constituting the kit. For this reason, the modes of use of the antibody and the antigen reagent and the respective reagents constituting the kit can be suitably chosen so as to be adapted to the assaying system employed and can be prepared by the use of known methods. In the following, the preparation methods of a solid phase antibody (antigen) and a labeled antibody (antigen) frequently used as the modes of use of the antibody and antigen reagents are described.

(1) Preparation of solid phase antibody:

Examples of materials of the carrier for immobilizing the antibody, are natural polymer derivative carriers such as cyanogen halide activated products of polysaccharides (e.g., cellulose, dextran, starch, dextrin, hydroxyethyl cellulose, p-aminophenoxyhydroxypropyl dextran, agarose and sephadex) (see Japanese Patent Publication No. 38543/1970), sodium meta-periodate activated products of polysaccharides (see Japanese Patent Laid-Open Publication No. 756/1983), aminoethylated or aminopropylated products of cellulose or derivatives thereof (see Japanese Patent Laid-Open Publication No. 42452/1984), mercaptized products of cellulose or derivatives thereof (see Japanese Patent Laid-Open Publication No. 80558/1983), cyanated products of polysaccharides (see Japanese Patent Publication No. 23031/1974), epichlorohydrin-p-aminophenol treated products of polysaccharides (see Japanese Patent Laid-Open Publication No. 158994/1979), diazotized products of polysaccharide derivatives containing aromatic amino groups (treated with dil. hydrochloric acid, sodium nitrite), cellulose carbonate derivatives, cellulose acetate, natural fiber (cotton, hemp, wool, etc.); polymer or copolymer carriers of ethylene, propylene, vinyl chloride, vinyl acetate, vinyl propionate, acrylic acid, acrylic acid ester, methacrylic acid, methacrylic acid ester, styrene, methylstyrene, butadiene, isoprene, acrylamide, acrylonitrile and methacrylonitrile; or these carriers having reactive functional groups such as amino, hydroxyl, carboxyl, sulfone, thiol, azide and isocyano introduced therein.

The shape of the carrier can be, for example, a tube, test plate, beads, disc, sphere, stick and latex.

The antibody to be immobilized on the carrier may be the antibody itself, or $F(ab')_2$, Fab' or Tab which is the active fragment of the antibody.

As the solid phase formation method, any of the physical adsorption method, the covalent bonding method, the crosslinking method and the entrapping method can be applied. As to details concerning these solid phase formation methods, the methods for the enzyme immobilization are applicable, and reference may be made to textbooks and reviews, for example, "Immobilized Enzyme" edited by Ichiro Chibata (published by Kabushiki Kaisha Kodansha, March 20, 1975), pp 9-75.

(2) Preparation of solid phase antigen:

As the materials and the shapes of the carrier for immobilizing the antigen, the same ones used for preparation of the solid phase antibody as described above can be used.

As the antigen to be immobilized on the carrier, a conjugate of endothelin and a protein can be used. As the conjugate of endothelin and protein, ones prepared according to the same methods as used for the preparation of immunogens as described above can be used.

As the solid phase formation method, any of the physical adsorption method, the covalent bonding method, the crosslinking method and the entrapping method is applicable similarly as in the case of the solid phase antibody.

(3) Preparation of labeled antibody:

Preparation of a labeled antibody can be practiced by using a known method established for the label or the label system employed. For example, in the case of radioisotope labeling, the chloramine T method, the lactoperoxidase method, the glucoseoxidase method, the binding method (Bolton-Hunter method), etc. can be used. In the case of enzyme labeling, the glutaraldehyde method, the periodic acid method, the maleimide method, the pyridyl-disulfide method, etc. can be used. Also, other than the method of labeling directly an enzyme on the antibody, it is also possible to use the indirect labeling method in which a biotin-labeled antibody and an enzyme-labeled avidin are employed.

(4) Preparation of labeled antigen:

Preparation of a labeled antigen can be practiced similarly by using a known method as in the case of labeled antibody. For example, in the case of radioisotope labeling, the chloramine T method, the lactoperoxidase method, the glucoseoxidase method, etc. can be applied. In the case of enzyme labeling, the method of binding indirectly through a spacer between the antigen and the enzyme, etc. can be applied. As the spacer, the binding agent as exemplified in the preparation of the immunogen as described above can be used.

A specific example of the kit of reagents for endothelin assay by the use of one or more of the solid phase antibody, the solid phase antigen, the labeled antibody and the labeled antigen as described in detail above will be described in detail by referring to an example of a kit for the enzyme immunoassay.

The kit [A] is an assay kit based on the competitive reaction method and composed mainly of the following reagents as the constituents:

a. solid phase antigen immobilized on a carrier
b. enzyme-labeled anti-endothelin monoclonal antibody
c. standard solution of endothelin
d. substrate solution necessary for enzyme activity assay
e. enzyme reaction stopping solution

The kit [B] is an assay kit based on the sandwich assay and composed mainly of the following reagents as the constituents:

a. solid phase anti-endothelin monoclonal antibody immobilized on a carrier
b. anti-endothelin polyclonal antibody (second antibody solution)
c. biotin-labeled anti-IgG antibody
d. enzyme-labeled avidin
e. standard solution of endothelin
f. substrate solution necessary for enzyme activity assay
g. enzyme reaction stopping solution

In addition to the constituents as mentioned above, if necessary, a dilution solution, coloring solution, washing solution, etc. can also be supplemented.

The above-mentioned kit [A] can be used according to, for example, the method as described below.

With the solid phase antigen, the standard solution of endothelin or the solution to be tested and the enzyme-labeled antibody are reacted at 0 to 40 °C for 0.5 to 5 hours. After separation of the liquid phase and the solid phase, the substrate solution is added into either one of the liquid phase and the solid phase to carry out the enzyme reaction, after which the enzyme reaction is stopped, and the optical intensity in the reaction mixture is measured.

The above-mentioned kit [B] can also be used according to, for example, the method described below.

With the solid phase antibody, the standard solution of endothelin or the solution to be tested is reacted at 0 to 40 °C for 0.5 to 5 hours. After washing the carrier, the second antibody solution is further added and the reaction is carried out at 0 to 40 °C for 0.5 to 5 hours. After washing the carrier, the biotin-labeled anti-IgG antibody is added to carry out the reaction, and then the enzyme-labeled avidin is added to carry out the reaction. After separation of the liquid phase from the solid phase, the substrate solution is added into either one of the liquid phase and the solid phase, and then the enzyme reaction is stopped. Thereafter absorbance is measured.

In the following, the present invention is described by referring to specific examples. In the following examples, as endothelin, a synthetic product having the same structure as that derived from the human was employed.

Example 1

Anti-endothelin antiserum

(1) Preparation of conjugate of endothelin and bovine serum albumin:
To a solution of 10 mg of bovine serum albumin (BSA) and 1-2 mg of endothelin in 1 ml of 0.1 M phosphate buffer (pH 7.0) was added dropwise under stirring 0.5 ml of 21 mM glutaraldehyde [dissolved in a 0.1 M phosphate buffer (pH 7.0)], and the reaction was carried out at room temperature for 24 hours. After the reaction, the reaction mixture was dialyzed against a 0.1 M phosphate buffer (pH 7.0) to remove unreacted substances to obtain a conjugate of endothelin and BSA.

The conjugate obtained was found to have an average of 5 molecules of endothelin bound per molecule of BSA.

(2) Preparation of anti-endothelin antiserum:
The above conjugate and complete Freund's adjuvant were mixed at 1:1 to form an emulsion, which was administered intraperitoneally into Balb/c mice (female, 6 weeks old) at 25 μg per mouse four times every three weeks. After elapse of 10 days after the final booster, the blood was sampled, left standing at 4°C for 16 hours, and the supernatant obtained by centrifugation at 3,000 rpm for 10 minutes was provided as the anti-endothelin antiserum.

The reactivity of the anti-endothelin antiserum with endothelin was examined by the ELISA method. More specifically, a conjugate of endothelin and mouse serum albumin (MSA) prepared under the same conditions as in the preparation of the conjugate of BSA and endothelin or MSA was dissolved in 0.1 M phosphate buffered saline (PBS) to 2 μg/ml, which solution was added into a 96-well microplate in an amount of 50 μl per well, left standing overnight at 4°C to coat the 96-well microplate with the conjugate of endothelin and MSA or MSA.

After the coating treatment, the wells were washed twice with PBS, a PBS containing 3% gelatin was added into each well, and the mixture was left standing at room temperature for 30 minutes to carry out the blocking treatment.

Into each well after the blocking treatment was added 50 μl of a diluted solution of the above-mentioned anti-endothelin antiserum, and the reaction was carried out at room temperature for one hour. After the reaction, the mixture was washed with PBS three times, and 50 μl of a biotinylated anti-mouse IgG antibody solution (Vector) was added, after which the reaction was carried out at room temperature for 1 hour.

Next, 50 μl of an avidin D-horse radish peroxidase solution (Vector) was added, and the reaction was carried out at room temperature for 30 minutes. After washing with PBS 3 times, 200 μl of a substrate solution (containing 4-aminoantipyrine (0.25 mg/ml), phenol (0.25 mg/ml) and 0.00125% hydrogen peroxide) was added to carry out the reaction at room temperature, and the absorbance of each well at 550 nm was measured by means of a 96-well microplate photometer.

The results obtained are shown in Fig. 1. As shown in Fig. 1, the anti-endothelin antiserum of the present invention was found to have specificity for endothelin and could be used for assay of endothelin in a test sample.

Example 2

Anti-endothelin antiserum

In the same manner as in Example 1 (1), a conjugate of egg albumin and endothelin was prepared. The conjugate obtained was found to have 5 molecules of endothelin bound per molecule of egg albumin.

A 1:1 emulsion of the conjugate and complete Freund's adjuvant was prepared, and rabbits were immunized with 0.5 mg of the emulsion per rabbit twice every three weeks. Ten days after the final booster, the blood was sampled, centrifuged at 3,000 rpm for 10 minutes and the supernatant was provided as the anti-endothelin antiserum.

The reactivity of the antiserum thus obtained with endothelin was assayed by the ELISA method described in Example 1, and the results are shown in Fig. 2. As shown in Fig. 2, the antiserum obtained from rabbits was found to have specificity for endothelin.

Example 3

Anti-endothelin antiserum

A conjugate of a synthetic peptide ($NH_2$-Arg-Cys-His-Leu-Asp-Ile-Ile-Trp-COOH) and BSA was prepared in the same manner as in Example 1. The conjugate obtained was found to have 6 molecules of the synthetic peptide per molecule of BSA.

Mice were immunized with the conjugate in the same manner as in Example 1, and the reactivity of the antiserum obtained with endothelin was examined by the ELISA method described in Example 1. The results are shown in Fig. 3. As shown in Fig. 3, an antiserum having specificity for endothelin could be prepared by the use of a synthetic peptide as the endothelin antigen.

Example 4

Anti-endothelin monoclonal antibody

(1) Preparation of anti-endothelin monoclonal antibody:
A solution of the endothelin-BSA conjugate of Example 1 in physiological saline (1 ml/l ml) and complete Freund's adjuvant were mixed at 1:1 to form an emulsion, which was administered in an amount of 50 μg intraperitoneally into Balb/c mouse (female, 6 weeks old) to effect the initial immunization.

After the initial immunization, booster was performed according to the same method several times every 2 weeks and then 50 μg of the emulsion was administered as the final booster into the tail vein of the mouse.

Spleen cells of the mouse were enucleated 3 days after the final booster, and washed with Eagle's minimum essential medium (MEM). On the other hand, mouse myeloma P3 x 63 Ag8U. I ($P_3U_1$) (ATCC CRL-1597) washed with MEM was mixed with the above-mentioned spleen cells at 10:1, and centrifuged. To the pellets obtained was added gradually 1 ml of an MEM solution containing 50% polyethylene glycol (PEG) 1000 to cause cell fusion. Further, the MEM solution was added to make up the whole amount to 10 ml, after which centrifugation was carried out. The pellets obtained were suspended in an RPMI 1640 medium containing 10% fetal calf serum (FCS) to 3 x $10^4$ cell/0.1 ml as $P_3U_1$, and apportioned into a 96-well microplate in an amount of 0.1 ml per well. One day later, 0.1 ml of HAT medium was added, and thereafter half amount of the medium was exchanged with new HAT medium every 3 to 4 days, and 100 μl of the supernatant in the well where growth of hybridoma was recognized was sampled and diluted with 200 μ1 of PBS. Into each of the 96-well microplate previously coated with MSA and the 96-well microplate coated with the endothelin-MSA conjugate (10 μg/ml) was added 50 μl of the diluted culture supernatant described above. Next, after addition of a biotinylated horse anti-mouse IgG antibody (Vector), according to the ELISA method using avidin D-horse radish peroxidase (Vector) as the avidin D-enzyme conjugate, and hydrogen peroxide and 4-aminoantipyrine-phenol as the substrate and a chromogenic agent, 21 strains of hybridomas producing antibodies which react with the endothelin-MSA conjugate but not with MSA were selected.

Next, according to the limiting dilution method, cloning of hybridomas was conducted. After cloning, the cells (hybridomas) were cultured to be increased in number and administered intraperitoneally into mice at 3 x $10^6$ cells per mouse after about one month after previous intraperitoneal administration of pristanė.

After 2 weeks, about 20 ml of ascites per mouse was sampled. After dilution of 40 ml of ascites (for 2 mice) with addition of equal amount of PBS, 80 ml of saturated ammonium sulfate solution was added, and the fractions precipitated under 50% saturated ammonium sulfate condition were collected by centrifugation. The precipitated fractions were dissolved by addition of about 10 ml of a 0.1 M Tris-hydrochloride buffer (pH 7.2), and the solution was dialyzed against the same buffer for 2 days.

Next, the antibody solution was added into a column (22 mm x 65 cm) filled with DE52 (Whatman), the fractions passed as such were collected, and then the fractions passed as such were added into a column (22 mm x 65 cm) filled with Ultrogel AcA44 (LKB), thereby obtaining a purified antibody.

(2) The immunological properties of the anti-endothelin monoclonal antibody:
For examination of the specificity of the monoclonal antibody obtained, first, each of the endothelin-MSA conjugate, the synthetic peptide-MSA conjugate and MSA was subjected to the coating treatment and

the blocking treatment in a 96-well microplate similarly as described in Example 1 to prepare a 96-well microplate having the antigen immobilized.

As the synthetic peptide, the same one as in Example 3 was employed.

The synthetic peptide has the same amino acids as the 7 residues at the C-terminus of the human endothelin, and the conjugate of said synthetic peptide and MSA was prepared according to the same method as in the preparation of the endothelin-BSA conjugate.

Into each well of the antigen-immobilized 96-well microplate thus prepared, 50 $\mu$l of the culture supernatant of each hybridoma strain was added, and the absorbance of each well at 492 nm was measured according to the same method as in the ELISA method as described in Example 1 except for the use of o-phenylenediamine in place of 4-aminoantipyrine and phenol as the substrate solution for comparative investigation of the specificities of the monoclonal antibodies produced by the respective hybridoma strains. The results are shown in Table 1. As Control, PBS containing 1% BSA was employed in place of the culture supernatant.

As is apparent from Table 1, monoclonal antibodies having two kinds of specificities, namely monoclonal antibodies exhibiting specificity for 7 residues at the C-terminus of endothelin (monoclonal antibodies produced by the respective hybridoma strains of the 2nd, 5th, 9th, 13th, 14th, 16th and 18-21th) and monoclonal antibodies exhibiting specificity for other sites than the 7 residues at the C-terminus of endothelin (monoclonal antibodies produced by the respective hybridoma strains of the 1st, 3rd, 4th, 6-8th, 10-12th, 15th and 17th) could be obtained.

(3) Sub-class of anti-endothelin monoclonal antibody:

The endothelin-MSA conjugate (2 $\mu$g/ml) was subjected to the coating and blocking treatments in a 96-well microplate similarly as in Example 1, and 50 $\mu$l of the culture supernatant of each hybridoma strain was added thereinto. The reaction was carried out at room temperature for one hour.

After the reaction, according to the ELISA method in which the rabbit anti-mouse Ig sub-class antibody (Zymed), the biotinylated goat anti-rabbit IgG antibody (Vector), the avidin D-horse radish peroxidase (Vector), the substrate solution (containing hydrogen peroxide and o-phenylenediamine), and the reaction stopping solution (2N sulfuric acid) were successively reacted, sub-classes of the monoclonal antibodies produced by the respective hybridomas were examined. The results are shown together in Table 1.

Table 1

| Hybridoma Strain | Absorbance (492 nm) | | | Sub-class |
|---|---|---|---|---|
| | Endothelin MSA Conjugate | Synthetic Peptide MSA Conjugate | MSA | |
| 1st | 1.396 | 0.132 | 0.262 | $\gamma_1\kappa$ |
| 2nd | 1.319 | 1.534 | 0.255 | $\gamma_1\kappa$ |
| 3rd | 1.532 | 0.126 | 0.312 | $\gamma_{2a}\kappa$ |
| 4th | 1.095 | 0.316 | 0.214 | $\gamma_{2a}\kappa$ |
| 5th | 1.210 | 1.506 | 0.229 | $\gamma_1\kappa$ |
| 6th | 1.489 | 0.138 | 0.228 | $\gamma_2\kappa$ |
| 7th | 1.329 | 0.098 | 0.236 | $\gamma_1\kappa$ |
| 8th | 1.222 | 0.107 | 0.261 | $\gamma_{2a}\kappa$ |
| 9th | 1.079 | 1.305 | 0.229 | ?$\kappa$ |
| 10th | 1.357 | 0.140 | 0.227 | $\gamma_{2b}\kappa$ |
| 11th | 1.137 | 0.107 | 0.236 | $\gamma_{2a}\kappa$ |
| 12th | 1.214 | 0.122 | 0.228 | $\gamma_{2a}\kappa$ |
| 13th | 0.880 | 0.825 | 0.228 | $\gamma_1\kappa$ |
| 14th | 1.246 | 1.375 | 0.275 | $\gamma_1\kappa$ |
| 15th | 1.243 | 0.306 | 0.262 | $\gamma_{2a}\kappa$ |
| 16th | 1.245 | 1.357 | 0.265 | $\gamma_1\kappa$ |
| 17th | 1.340 | 0.101 | 0.274 | ?$\kappa$ |
| 18th | 1.269 | 1.454 | 0.299 | $\gamma_1\kappa$ |
| 19th | 1.212 | 1.122 | 0.293 | $\gamma_1\kappa$ |
| 20th | 1.381 | 1.403 | 0.305 | $\gamma_1\kappa$ |
| 21st | 1.461 | 1.161 | 0.309 | $\gamma_1\kappa$ |
| Control | 0.204 | 0.154 | 0.259 | |

(4) Neutralization action of anti-endothelin monoclonal antibody:

The neutralization action of endothelin with anti-endothelin monoclonal antibody was investigated in an in vivo system.

Into femoral artery and carotid of Wister Kyoto rat (WKY, male, 8-10 weeks old, weighing 300 to 350 g) was inserted a cannula.

Drugs (angiotensin II and endothelin) were injected through the cannula inserted into carotid under no anesthesia and no restraint condition, and the actions of the drugs on blood pressure and heart rate were measured by a pressure transducer connected to the cannula inserted into femoral artery. First, the change in blood pressure or heart rate when 300 pmol/kg of angiotensin II, 300 pmol/kg of endothelin and 1 nmol/kg of endothelin were injected is shown in Fig. 4(a).

Next, the changes in blood pressure and heart rate when the same drugs as mentioned above were injected in the same doses at the points of elapse of 5 to 10 minutes after intravenous injection of 8 mg/kg of the monoclonal antibody produced by the 20th hybridoma strain are shown in Fig. 4(b).

As is apparent from Fig. 4, when no anti-endothelin antibody of the present invention is administered, although changes in blood pressure and heart rate may be observed (Fig. 4(a), (a-2) and (a-3)), there is no such change observed at all when the anti-endothelin antibody is administered before administration of endothelin (Fig. 4(b), (b-2) and (b-3)), whereby it is confirmed that the anti-endothelin monoclonal antibody of the present invention neutralizes (inhibits) completely the activity of endothelin. Also, as shown in Fig. 4 (a-1) and (b-1), the changes in blood pressure and heart rate by angiotensin II are observed regardless of administration of the antibody of the present invention, whereby it is also confirmed that the antibody of the present invention neutralizes specifically endothelin.

Example 5

Assay of endothelin (competitive reaction)

The endothelin-MSA conjugate (0.6 μg/ml) was added into a 96-well microplate in an amount of 50 μl per well and left standing at 4°C overnight to coat the 96-well microplate with the endothelin-MSA conjugate, thus providing a solid phase antigen.

The culture supernatants of the hybridoma strains of the 16th, 17th and 20th hybridoma strains in Example 4 were suitably diluted with PBS containing 1% BSA (diluted to 30 - 300-fold) to provide antibody solutions.

Endothelin and the synthetic peptide as described in Example 3 were diluted stepwise with PBS containing 1% BSA respectively to concentrations in the range of from 0 to 2560 pM to provide standard antigen solutions.

By the use of the solid phase antigen, the antibody solution and the standard antigen solution, calibration curves were prepared according to the procedure indicated below.

(1) Each 30 μl of the antibody solution and the antigen solution are mixed, and the reaction is carried out at room temperature for 30 minutes.

(2) 50 μl of the reaction mixture is added to the solid phase antigen, and the reaction is carried out at room temperature for 1 hour.

(3) After washing twice with PBS, 50 μl of the biotinylated horse anti-mouse IgG (Vector) solution is added, and the reaction is carried out at room temperature for one hour.

(4) After washing twice with PBS, 50 μl of an avidin D-horse radish peroxidase (Vector) solution is added, and the reaction is carried out at room temperature for 15 minutes.

(5) After washing four times with PBS, 100 μl of a substrate solution [0.1 M citrate buffer (pH 5.0) containing o-phenylenediamine (0.2 mg/ml) and aqueous hydrogen peroxide (0.005 %)] is added to cause color formation, and the reaction is stopped by addition of 100 μl of 2 N sulfuric acid solution, followed by measurement of absorbance at 492 nm.

The calibration curves thus prepared are shown in Figs. 5, 6, and 7. As is apparent from Figs. 5, 6, and 7, it has been clarified that endothelin of 40 pM can be assayed in the above-described method. Also, since other portions than the 7 residues at the C-terminus of endothelin can be specifically assayed by the use of the antibody obtained from the 17th hybridoma strain, it has been shown that the hydrolyzates of endothelin can also be assayed.

Also, the respective reagents comprising the solid phase antigen, the antibody solution, the standard antigen solution, the biotinylated horse anti-mouse IgG antibody solution, the avidin D-horse radish peroxidase solution, the substrate solution and the 2 N sulfuric acid solution (reaction stopping solution) were packed in one small box to provide a kit for assay of endothelin.

Example 6

Assay of endothelin (the sandwich assay)

As the solid phase antibody, the monoclonal antibody coated on a 96-well microplate obtained by

adding the monoclonal antibody produced by the first hybridoma strain (50 μg/ml) into the 96-well microplate in an amount of 50 μl per well and then leaving the wells standing at 4° C overnight, was employed.

As the standard antigen solution, a solution obtained by diluting stepwise endothelin with a 0.1 M Tris-hydrochloride buffer (pH 8.0) containing 0.5 M sodium chloride, 10% BS (bovine serum) and 5% tradirol to a concentration range of 0 to 12.8 (ng/ml) was employed.

As the second antibody solution, a solution of the monoclonal antibody which was produced by the 5th hybridoma strain, then biotinylated in the conventional manner and dissolved in a 0.1 M phosphate buffer (pH 7.0) contaiing 1% BSA to a concentration of 2 μg/ml was employed.

The assay was carried out according to the following procedure.

(1) Into the 96-well microplate coated with the antibody, the antigen solution is added in an amount of 50 μl per well, and the reaction is carried out at room temperature for 2 hours.

(2) After washing with PBS, the second antibody solution is added in an amount of 50 μl per well, and the reaction is carried out at room temperature for 1 hour.

(3) After washing with PBS, the avidin D-horse radish peroxidase (Vector) solution is added in an amount of 50 μl per well, and the reaction is carried out at room temperature for 30 minutes.

(4) After washing with PBS, the substrate solution [0.1 M citrate buffer (pH 5.0) containing o-phenylenediamine (0.2 mg/ml) and aqueous hydrogen peroxide (0.005%)] is added in an amount of 100 μl per well to cause color formation, and the reaction is stopped by addition of 100 μl of 2N sulfuric acid solution, followed by measurement of absorbance at 492 nm.

The calibration curve thus obtained is shown in Fig. 8. As is apparent from Fig. 8, it has been confirmed that, by applying the antibody of the present invention to the sandwich method, assay up to 0.2 ng/ml (80 pM) of endothelin is possible.

Also, the respective reagents comprising the solid phase antibody, the standard antigen solution, the second antibody solution, the avidin D-horse radish peroxidase, the substrate solution, and the 2N sulfuric acid solution (reaction stopping solution) were packed in one small box to provide a kit for assay of endothelin.

## UTILIZABILITY IN INDUSTRY

Since the antibody of the present invention has specificity for endothelin, it is useful as an antibody to be used in assaying immunologically endothelin in blood. Particularly, the anti-endothelin monoclonal antibody has the following characteristics as compared with anti-endothelin antiserum and is particularly useful.

(1) As compared with antiserum, the reaction specificity and affinity are limited.

For example, the monoclonal antibodies shown in Example 4 exhibit specificity at either the C-terminus of endothelin or other sites than the C-terminus, and by the use of these two kinds of monoclonal antibodies, endothelin, itself as a matter of course, and also fragments of endothelin hydrolyzed in blood can be specifically assayed.

(2) As compared with antiserum, by establishing a hybridoma which produces the monoclonal antibody, the monoclonal antibody of constant quality can be supplied stably.

(3) According to the competitive reaction method, 40 pM of endothelin can be assayed, whereby an assay system of extremely high sensitivity can be assembled. Also, as shown in Example 6, according to the sandwich assay using two kinds of monoclonal antibodies with different specificities, an assay system which can assay 80 pM of endothelin can be assembled.

(4) When the anti-endothelin monoclonal antibody of the present invention is administered into a living body, the action of endothelin can be neutralized (see (4) of Example 4), whereby a possibility as a pharmaceutical can be provided.

Also, as shown in Example 3, by the use of a synthetic peptide, antiserum and monoclonal antibody which react with endothelin can be prepared. This means that the time and the labor for synthesizing chemically the whole endothelin molecule to be used as the antigen can be shortened to a great extent.

## Claims

**1.** An antibody having specificity for endothelin.

**2.** An antibody according to claim 1, wherein the antibody is a polyclonal antibody.

**3.** An antibody according to claim 1, wherein the antibody is a monoclonal antibody.

**4.** An antibody according to any of claims 1 to 3, wherein the endothelin is derived from a human or pig.

**5.** An antiserum containing as the main ingredient thereof an antibody having specificity for endothelin.

**6.** An antiserum according to claim 5, wherein the endothelin is derived from a human or pig.

**7.** An antibody having specificity for endothelin represented by the following formula [I]:

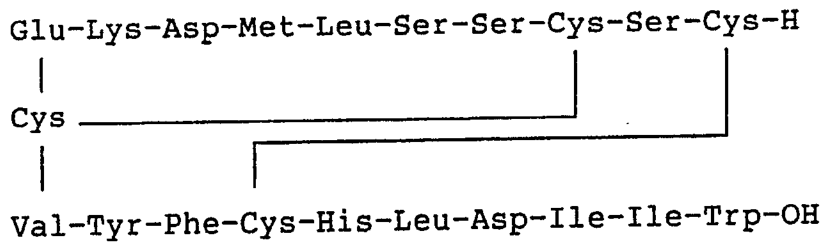

[I]

[wherein Cys represents L-cysteine; Ser, L-serine; Leu, L-leucine; Met, L-methionine; Asp, L-aspartic acid; Lys, L-lysine; Glu, L-glutamic acid; Val, L-valine; Tyr, L-tyrosine; Phe, L-phenylalanine; His, L-histidine; Ile, L-isoleucine; Trp, L-tryptophan].

**8.** An antibody according to claim 7, wherein the antibody is a polyclonal antibody.

**9.** An antibody according to claim 7, wherein the antibody is a monoclonal antibody.

**10.** A method for producing an antibody having specificity for endothelin, which comprises using a conjugate having endothelin bound to a carrier as an immunogen.

**11.** A method for immunologically assaying endothelin in a test sample, which comprises using an antibody having specificity for endothelin as an antibody reagent.

**12.** A method according to claim 11, wherein the antibody is a polyclonal antibody.

**13.** A method according to claim 11, wherein the antibody is a monoclonal antibody.

**14.** A kit for assay of endothelin comprising as the principal constituent thereof an antibody having specificity for endothelin.

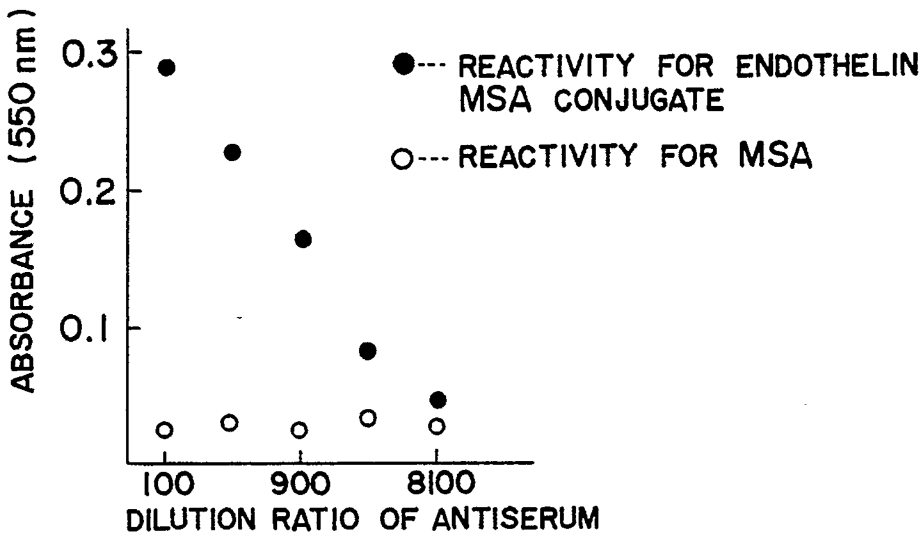


FIG. 1

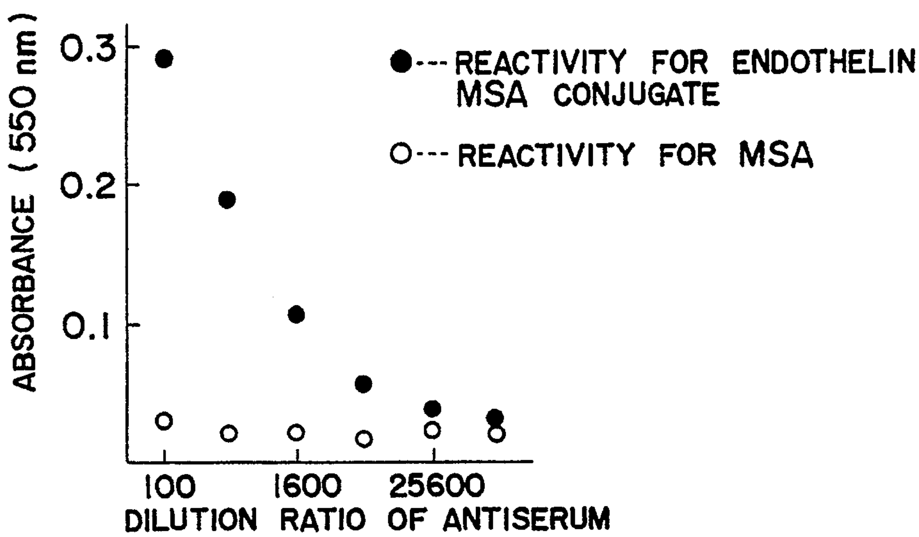


FIG. 2

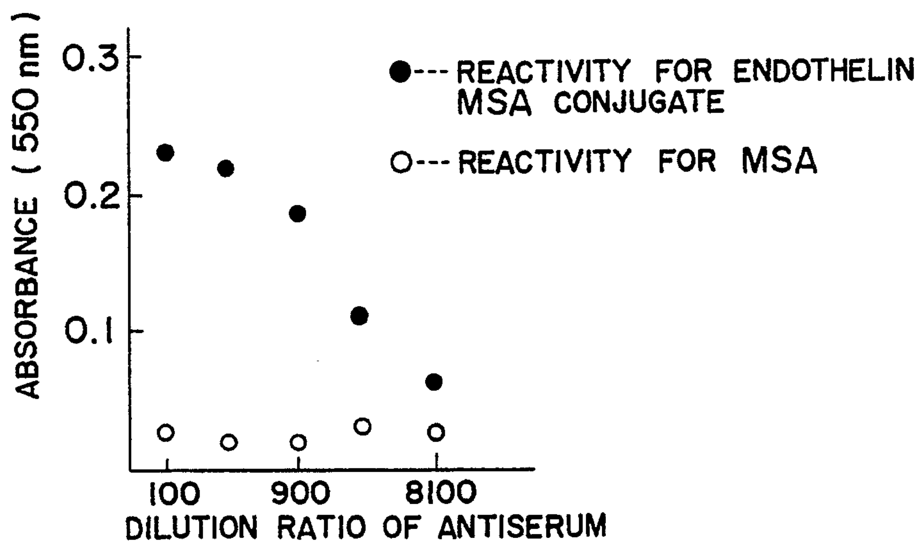


FIG. 3

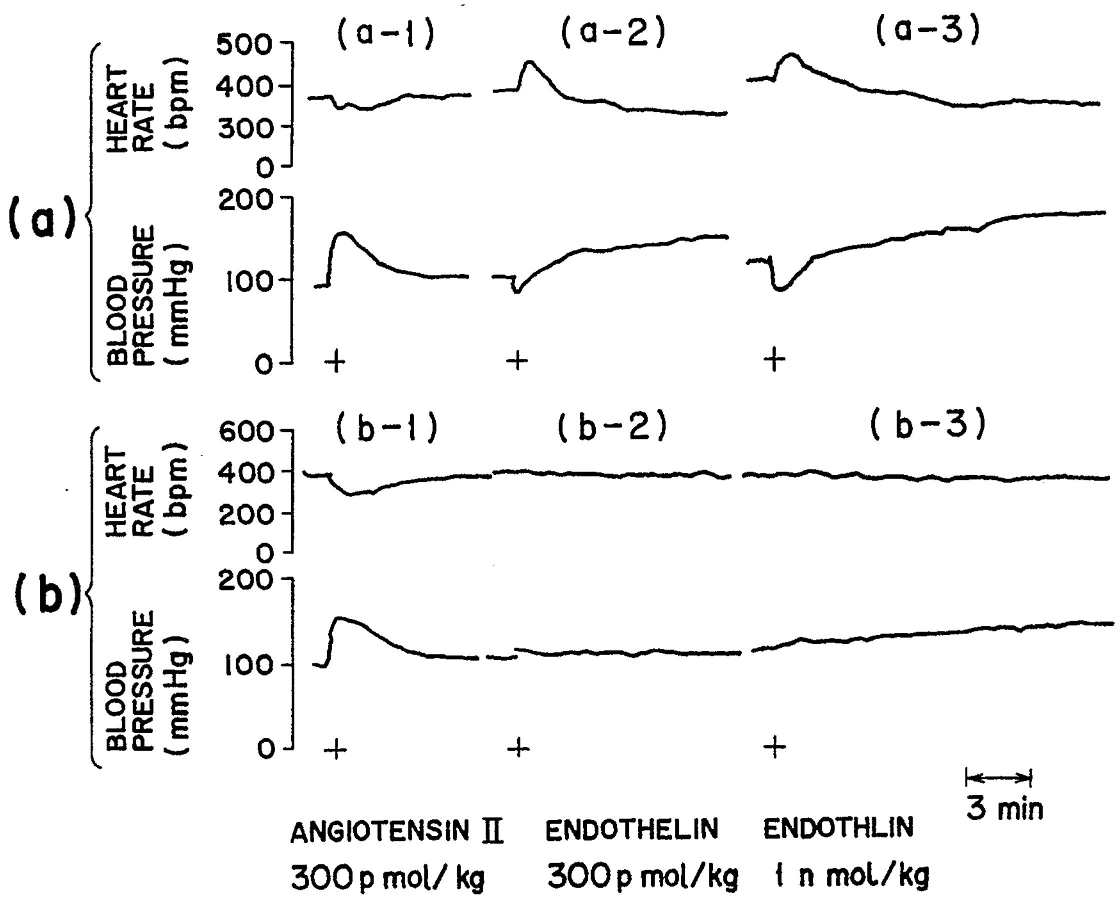
(a-1)
(a-2)
(a-3)
(a)
HEART RATE (bpm)
500
400
300
0
BLOOD PRESSURE (mmHg)
200
100
0
(b-1)
(b-2)
(b-3)
(b)
HEART RATE (bpm)
600
400
200
0
BLOOD PRESSURE (mmHg)
200
100
0
3 min
ANGIOTENSIN II
300 p mol/kg
ENDOTHELIN
300 p mol/kg
ENDOTHLIN
1 n mol/kg

FIG. 4

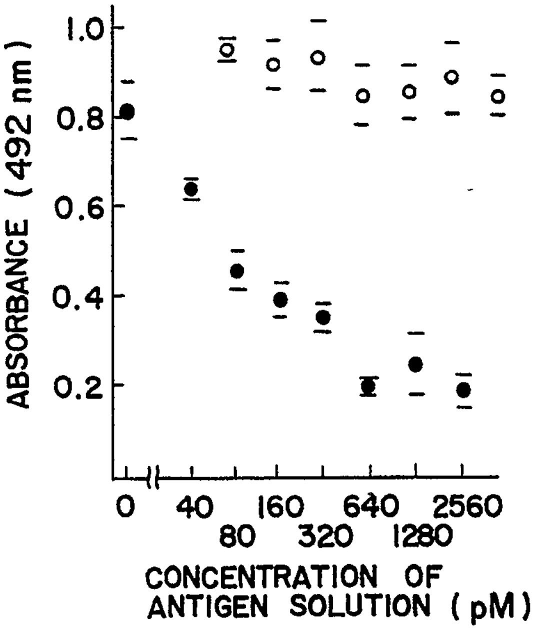


FIG. 5

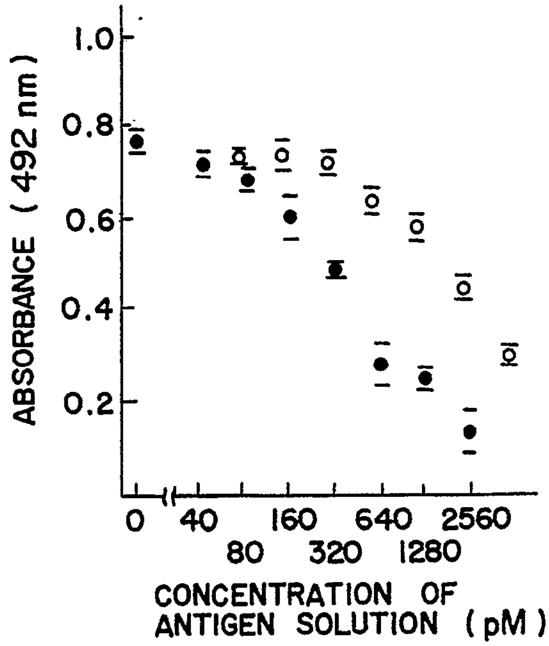


FIG. 6

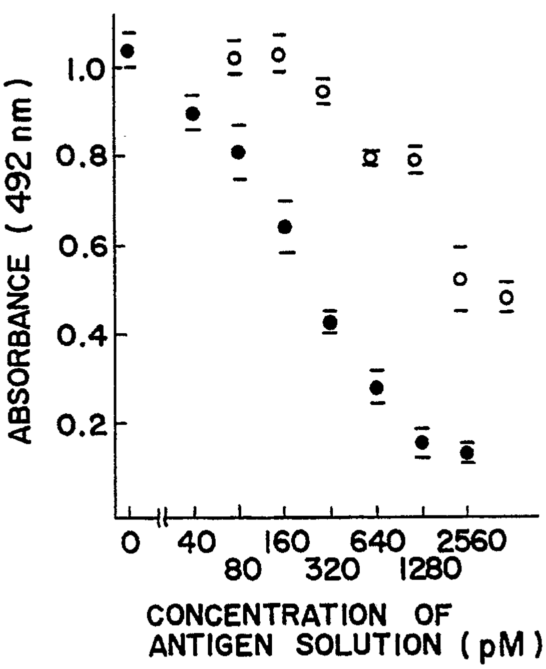


FIG. 7

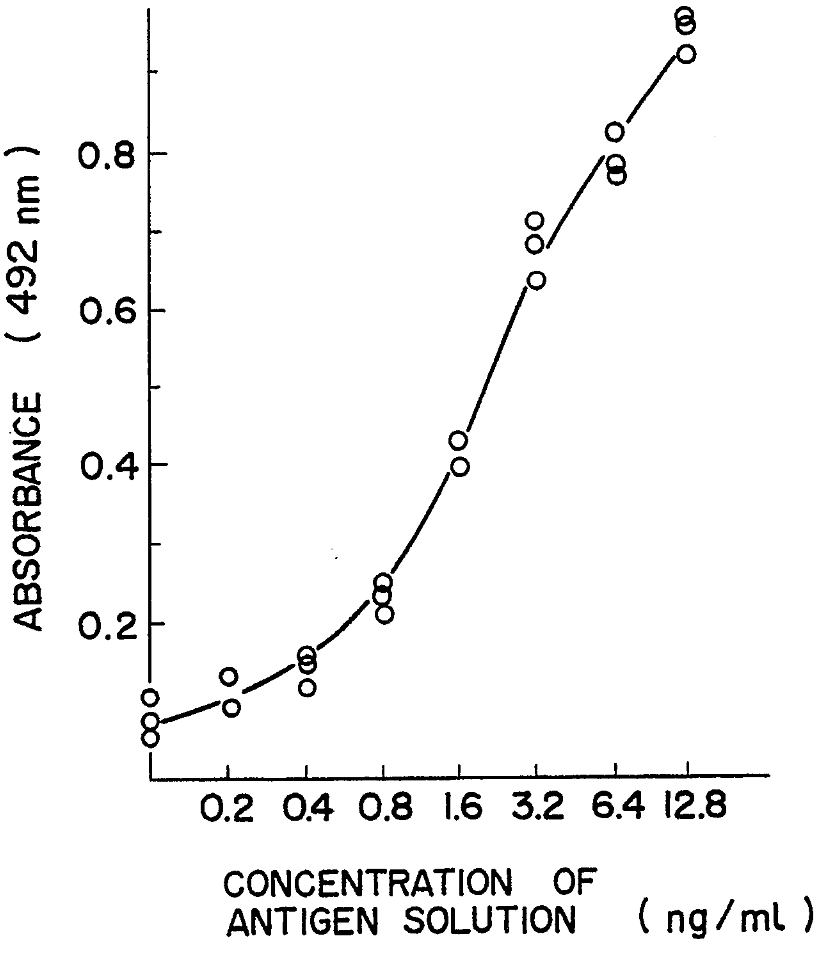
ABSORBANCE ( 492 nm )
0.8
0.6
0.4
0.2
0.2 0.4 0.8 1.6 3.2 6.4 12.8
CONCENTRATION OF
ANTIGEN SOLUTION ( ng/ml )

F I G. 8

# INTERNATIONAL SEARCH REPORT

International Application No PCT/JP89/00455

**I. CLASSIFICATION OF SUBJECT MATTER** (if several classification symbols apply, indicate all) [6]

According to International Patent Classification (IPC) or to both National Classification and IPC

Int. $Cl^4$ G01N33/53, G01N33/577

**II. FIELDS SEARCHED**

Minimum Documentation Searched [7]

| Classification System | Classification Symbols |
|---|---|
| IPC | G01N33/53, G01N33/577 |

Documentation Searched other than Minimum Documentation to the Extent that such Documents are Included in the Fields Searched [8]

Jitsuyo Shinan Koho 1971-1989
Kokai Jitsuyo Shinan Koho 1971-1989

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| P | Journal of Immunological Methods, Vol. 118, No. 2, (1989), [A sensitive sandwich-enzyme immunoassay for human endothelin], Nobuhiro Suzuki et al. P. 245-250 | 1-14 |
| Y | Nature, Vol. 332, No. 6163 (1988), Masashi Yanagisawa et al. [A novel potent vasoconstrictor peptide produced by vasular endothelial cells], P. 411-415 | 1-14 |
| Y | FEBS LETTERS, Vol. 231, No. 2, (1988), Yasuaki Itoh et al., [Cloning and sequence analysis of c DNA encoding the precursor of a human endothelium-derived vasoconstrictor peptide, endothelin: identity of human and porcine endothelin], P. 440-444 | 1-14 |
| Y | JP, A, 59-163565 (Toray Industries, Inc.) (Family: none) | 1-14 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 10, 1989 (10.07.89) | July 24, 1989 (24.07.89) |
| **International Searching Authority** | **Signature of Authorized Officer** |
| Japanese Patent Office | |